# EUROPÄISCHE PATENTSCHRIFT

(11) **EP 4 114 192 B1**
(45) Veröffentlichungstag und Bekanntmachung des Hinweises auf die Patenterteilung: **20.12.2023**
(21) Anmeldenummer: 21708196.7
(22) Anmeldetag: 24.02.2021
(51) Int. Cl.: A23K 20/00, A23K 50/30, A23K 20/105, A23K 10/30

(54) **VERFAHREN ZUR VERMINDERUNG VON VERHALTENSSTÖRUNGEN BEI SCHWEINEN**
METHOD FOR REDUCTION OF BEHAVIORAL DISORDERS IN PIGS
PROCÉDÉ DE RÉDUCTION DES TROUBLES DU COMPORTEMENT CHEZ LES PORCS

(30) Priorität: 04.03.2020 DE 102020105769
(43) Veröffentlichungstag der Anmeldung: 11.01.2023
(73) Patentinhaber: Alzchem Trostberg GmbH, 83308 Trostberg (DE)
(72) Erfinder: SANS, Jürgen, 83308 Trostberg (DE)
(74) Vertreter: Weickmann & Weickmann PartmbB
(86) Internationale Anmeldenummer: PCT/EP2021/054580
(87) Internationale Veröffentlichungsnummer: WO 2021/175677

(56) Entgegenhaltungen:
- WO-A1-2005/120246
- CN-A- 103 859 208
- CN-A- 105 076 769

## Beschreibung

Die vorliegende Erfindung betrifft die nicht-therapeutische Verwendung von Guanidinoessigsäure sowie ein nicht-therapeutisches Verfahren zur Behandlung der Caudophagie bei Schweinen.

Das Hausschwein (lat. Sus scrofa domesticus, nachfolgend lediglich Schwein genannt) ist die domestizierte Form des Wildschweins und bildet mit ihm eine einzige Art. Es gehört damit zur Familie der Echten Schweine. Das Schwein ist eines der ältesten domestizierten Haustiere in der menschlichen Zivilisationsgeschichte und wird seit vermutlich 9000 Jahren zur Fleischerzeugung gehalten. In Europa und weiteren Regionen der Welt zählt Schweinefleisch zu der am häufigsten gegessenen Fleischsorte.

WO 2005/120246 A1 beschreibt Guanidinoessigsäure als Futtermittelzusatz. CN 103859208 beschreibt einen Futtermittelzusatz für Ferkel. CN 105076769 beschreibt ein Futteradditiv für Schweine.

Eine häufig auftretende Verhaltensstörung bei Schweinen während ihrer Aufzucht und Mast ist das Schwanzbeißen, wissenschaftlich auch Caudophagie genannt. Diese Verhaltensstörung hat eine große wirtschaftliche Bedeutung [vgl. The EFSA Journal (2007) 611, 1-13: The risks associate with tail biting in pigs and possible means to reduce the need for tail docking considering the different housing and husbandry systems]. Zu dem Phänomen Verhaltensstörungen gehören neben dem Schwanzbeißen auch Verletzungen an Ohren, Flanken oder den Extremitäten.

Durch die über Jahrhunderte währende Zucht wurden die Schweine agiler, aber auch sensibler und empfindlicher gegenüber Umwelteinflüssen. Des Weiteren wurde der strohlose Spaltboden eingeführt. Beide Faktoren verstärkten die Problematik des Schwanzbeißens [vgl. Sambraus, H. (1985): Mouth-based anomalous syndromes, In: Ethology of arm animals. Worldanimal science A5. A.F. Fraser: Elsevier 1985, 391-421].

Es können sich Ertragseinbußen durch verminderte Zunahme an Gewicht (Mastleistung) ergeben. Aufgrund von Infektionen kann auch ein Verwerfen des Schlachtkörpers notwendig werden. Generell führt Schwanzbeißen zu einer Reduzierung des Wohlbefindens der Tiere.

Als eine mögliche vorbeugende Maßnahme zur Vermeidung des Schwanzbeißens werden die Schwänze von Ferkeln kupiert, um somit gesundheitliche Schäden (wie zum Beispiel Schwanznekrosen) in der Ferkelaufzucht- und Mastphase zu verringern. Das Kupieren stellt die sicherste Methode dar, um das Schwanzbeißen zu vermeiden [Moinard, C.; Mendel, M.; Nicol, C.; Green, L. (2003): A case control study of on-farm risk factors for tail biting in pigs. Applied Animal Behaviour Science 81(4), 333-355], [Hunter, E; Jones, T.; Guise, H.; Penny, R.; Hoste, S. (2001): The relationship between tail biting in Pigs, Docking Procedure and other Management Practices. The Veterinary Journal 161, 72-79].

Aus Tierschutzgründen sollte diese Maßnahme jedoch nur in Ausnahmefällen durchgeführt werden. Nach einer EU Richtlinie 2008/120/EG des Rates über Mindestanforderungen für den Schutz von Schweinen ist das routinemäßige Kupieren der Ferkelschwänze verboten. Dennoch werden in Deutschland eine hohe Zahl von Ferkeln kupiert. Die Betriebe verfügen über eine Bescheinigung des betreuenden Tierarztes, die die Unerlässlichkeit des Schwanzkürzens bestätigt [vgl. Knoop, S.; Schrade, H. (2010): Problematik Schwanzbeißen/Schwänze kupieren bei Schweinen. Bildungs- und Wissenszentrum Boxberg, Landesanstalt für Schweinezucht].

Durch verschiedene Maßnahmen soll das Schwanzbeißen bei Schweinen minimiert werden können. Hierfür wurde eine Verhaltensempfehlung herausgebracht [vgl. Meyer, E. (2019): Checkliste zur Vermeidung von Verhaltensstörungen, URL: https://www.tsk-sachsen.de/index.php/tiergesundheitsdienste/schweinegesundheit/veroeffent lichungenschweine/232-ueberarbeitete-checkliste-zur-vermeidung-von-verhaltensstoerungen]. Genannt werden als Maßnahmen für die Haltungsbedingungen die Separierung von Problemtieren, die Haltungsdichte, die Sortierung, die Lichtgestaltung, die Buchtenstruktur. Weitere Maßnahmen zielen auf das Stallklima, Futter und Wasser, Beschäftigung und Tiergesundheit ab.

Nachteilig an diesem Verfahren ist jedoch, dass durch eine niedrige Haltungsdichte zwar die Tier-Tier-Kontakte verringert werden, parallel hierzu aber die Produktionsrate pro m² sinkt. Des Weiteren sind die aufgeführten empfohlenen Maßnahmen sehr aufwendig in der täglichen Umsetzung und es sind erhebliche Investitionen zu tätigen.

Schwanzentzündungen und Nekrosen müssen vermieden werden, um einen intakten Ringelschwanz zu erhalten. Endotoxine können für die Entzündungsprozesse verantwortlich sein. Diese Toxine kommen primär aus dem Darm. Mykotoxine (pilzlicher Herkunft) können den Abbau von Endotoxinen beeinflussen, da Mykotoxine die Darmbarriere schwächen und das Immunsystem und den Stoffwechsel negativ beeinflussen [vgl. Quanz, G. (22.10.2018) Pressemitteilung LWZ Eichhoff: Gesunder Schwanz durch Futterzusatzstoffe?, URL: https://www.bwagrar.de/Aktuelles/Gesunder-Schwanz-durch-Futterzusatzstoffe,QUIEPTU5NDUwMDkmTUIEPTUxNiQ0.html].

Um die Endo- und Mykotoxine unschädlich zu machen, werden seitens der Hersteller für veterinärmedizinische Mittel verschiedenste Präparate angeboten. Diese Präparate haben jedoch meist auch Nebenwirkungen, die seitens der Mastbetriebe unerwünscht sind.

Das Schwanzbeißen kann in allen Lebensphasen, vermehrt in konventionell, aber auch in ökologisch wirtschaftenden Betrieben auftreten [vgl. Goßmann, J.; Hoy, S. (2014): Graspellets kontra Schwanzbeißen, dlz primus schwein 10/2015, 42-45]. Es zeigte sich, dass Tiere, die zusätzlich zu ihrer Standardfutterration mit 5 % Wiesengraspellets gefüttert wurden, 11 % bzw. 13 % weniger zu Schwanzbeißen neigten. Mit dem Schwanzbeißen einher geht der Durchfall der Tiere. Eine Reduktion des Schwanzbeißens reduziert damit auch den Durchfall. Nachteilig an dem beschriebenen Verfahren ist, dass ein relativ bescheidener Erfolg durch Zusatz von relativ großen Mengen an Graspellets erzielt wurde.

Weiterhin ist das Schwanzbeißen nur ein Phänomen einer Verhaltensstörung. Durch das Kupieren der Schwänze verschwindet nicht die Aggressivität / Verhaltensstörung der Tiere. Es wird den Tieren lediglich eine Möglichkeit genommen, ihre Aggressivität auszuleben. Daher wird sich, wie oben erwähnt, die Aggression auf andere Körperteile wie auf Ohren, Flanken oder die Extremitäten richten.

Es besteht daher ein Bedarf an einer einfach umzusetzenden und kostengünstigen Methode, um die Verhaltensstörungen von Schweinen und das damit einhergehende aggressive Verhalten zu vermindern.

Gelöst wird diese Aufgabe durch die Verabreichung von Guanidinoessigsäure. Somit ist gemäß einer ersten Ausführung die nicht-therapeutische Verwendung von Guanidinoessigsäure als Mittel zur Verminderung der Aggressivität von Schweinen, insbesondere als nicht-therapeutisches Mittel zur Verminderung der Aggressivität von Schweinen, und/oder als Mittel zur Behandlung der Caudophagie bei Schweinen, insbesondere als nicht-therapeutisches Mittel zur Behandlung der Caudophagie bei Schweinen, Gegenstand der vorliegenden Erfindung.

Gemäß einer weiteren Ausführung ist zudem ein nicht-therapeutisches Verfahren zur Behandlung der Caudophagie bei Schweinen Gegenstand der Erfindung, indem den Schweinen Guanidinoessigsäure als Mittel zur Verminderung der Aggressivität, insbesondere als nicht-therapeutisches Mittel zur Verminderung der Aggressivität, verabreicht wird.

Überraschender Weise hat sich gezeigt, dass Guanidinoessigsäure geeignet ist die Aggressivität von Schweinen zu verringern. Vollkommen überraschend hat sich gezeigt, dass Schweine ein sehr viel friedvolleres Verhalten untereinander zeigen, wenn ihnen Guanidinoessigsäure oral verabreicht wird. In grundlegenden Untersuchungen hat sich gezeigt, dass Schweine, denen Guanidinoessigsäure verabreicht wurde, im Vergleich zu einer unbehandelten Kontrollgruppe weniger Beißattacken auf mit ihnen zusammenlebende Artgenossen ausüben. Die so behandelten Schweine zeigten im Vergleich zur unbehandelten Kontrollgruppe weniger Verhaltensauffälligkeiten in Form eines Schwanzbeißens (Caudophagie). Somit kann Guanidinoessigsäure effizient zur Behandlung und prophylaktischen Behandlung der Caudophagie bei Schweinen eingesetzt werden.

Guanidinoessigsäure (syn. Glycocyamin, N-Guanylglycin, N-Amidinoglycin; C₃H₇N₃O₂; CAS-Nr. 352-97-6, nachfolgend auch GAA genannt) gemäß der vorliegenden Erfindung bedeutet die Verbindung an sich als Feststoff, die Verbindung gelöst in einem geeigneten Lösungsmittel, insbesondere in Wasser sowie ihre Salze und hierbei besonders das Na-, K-, Mg-, und Ca-Salz. Guanidinoessigsäure ist seit einiger Zeit als Futtermittelzusatz auf dem Markt erhältlich und ist in der Geflügelmast zugelassen. Vielfältige Studien haben u.a. gezeigt, dass Guanidinoessigsäure eine Verbesserung der Futteraufnahme und eine Steigerung der Mastleistung bei Geflügel bewirkt.

Das "Schwanzbeißen" ist eine Verhaltensstörung von einzelnen Schweinen, die ihren Gruppenmitgliedern in die Ringelschwänze beißen. Diese Verhaltensstörung wird in Fachkreisen auch als Caudophagie bezeichnet. Die Ursache konnte durch die Wissenschaft noch nicht vollständig geklärt werden. Weitgehend sicher ist jedoch, dass es sich um ein multifaktorielles Geschehen handelt. Auslöser dieses Verhaltens sind meist verschiedene Faktoren gleichzeitig. Insbesondere können als Auslöser oder Ursache für diese Verhaltensstörung u.a. das Stallklima, d.h. die Temperatur, die Temperaturschwankungen, die Schadgase und die Luftfeuchtigkeit, die Haltungsbedingungen, d.h. die Besatzdichte, die Buchtenstruktur, die absolute Größe eines Bestandes, die Fressstellen pro Tier, die Fütterungstechnik, Futtermenge und Futterzusammensetzung, sowie die allgemeine Tiergesundheit der Schweine und fehlendes Beschäftigungsmaterial in Frage kommen.

Unter einer Behandlung soll gemäß der vorliegenden Erfindung sowohl die Behandlung einer akuten Auffälligkeit als auch eine prophylaktische Behandlung verstanden sein.

Schweine sind von Natur aus gesellige Tiere, die in mehr oder weniger größeren Gruppen zusammenleben. Auch in der Natur lassen sich aggressive Verhaltensweisen der Tiere untereinander beobachten. Werden Schweine jedoch in Stallungen gehalten, so nehmen auffällige Verhaltensweisen wie beispielsweise Aggressivität zu.

Mit den der Erfindung zugrundeliegenden Untersuchungen hat sich nunmehr gezeigt, dass Guanidinoessigsäure in sehr wirkungsvoller Art und Weise während der Aufzucht und der Mast von Schweinen eingesetzt werden kann. Insbesondere in der Stallhaltung von Schweinen kann Guanidinoessigsäure als Mittel, insbesondere als nicht-therapeutisches Mittel, zur Verminderung der Aggressivität der Schweine, und/oder als Mittel, insbesondere als nicht-therapeutisches Mittel, zur Behandlung der Caudophagie bei Schweinen, eingesetzt werden. Die Verminderung der Aggressivität zeigt sich u.a. in Form von weniger Beißattacken in die Flanken oder andere Körperteile wie beispielsweise die Ohren der mit ihnen zusammenlebenden Artgenossen.

Somit ist insbesondere auch die nicht-therapeutische Verwendung der Guanidinoessigsäure sowie ein nicht-therapeutisches Verfahren zur Behandlung der Caudophagie bei Schweinen Gegenstand der vorliegenden Erfindung, in dem Guanidinoessigsäure als Mittel, insbesondere als nicht-therapeutisches Mittel während der Aufzucht oder Mast der Schweine verabreicht wird.

Verhaltensstörungen in Form einer erhöhten Aggressivität und insbesondere in Form des Schwanzbeißen treten weniger bei Ferkeln oder Jungschweinen bis zu einem Alter von 40 Lebenstagen auf. Diese Verhaltensstörungen werden häufig ab einem Lebensalter von 60 Tagen vermehrt beobachtet. Eine gezielte Verabreichung von Guanidinoessigsäure gemäß der vorliegenden Erfindung ab dem 90. Lebenstag der Schweine konnte im Vergleich zu unbehandelten Kontrollgruppen eine deutliche Verminderung der Aggressivität und der hiermit einhergehenden Verletzungen in der Folgezeit bewirken. Letztendlich konnte somit auch eine vorbeugende Behandlung der Schweine mit entzündungshemmenden Arzneimitteln oder Desinfektionsmitteln reduziert werden.

Somit ist auch eine nicht-therapeutische Verwendung oder ein nicht-therapeutisches Verfahren Gegenstand der vorliegenden Erfindung, in der bzw. indem das nicht-therapeutische Mittel ab dem 60. Lebenstag, insbesondere ab den 70. Lebenstag, bevorzugt ab dem 80. Lebenstag und ganz besonders bevorzugt ab dem 90. Lebenstag der Schweine verabreicht wird.

Verhaltensstörungen in Form einer erhöhten Aggressivität und insbesondere in Form des Schwanzbeißen treten sowohl in der konventionellen als auch in der ökologischen Viehhaltung auf. Ein erhöhtes Platzangebot beispielsweise in Form eines Freilaufs kann diesen Verhaltensstörungen entgegenwirken, jedoch nicht vollständig beheben. Andererseits treten diese Verhaltensstörungen vermehrt in der Stallhaltung auf. Mit den der Erfindung zugrundeliegenden Untersuchungen konnte gezeigt werden, dass die Verabreichung von Guanidinoessigsäure bei Schweinen, denen ein Platzangebot von 0,75 bis 2,25 m²/Tier zu Verfügung stand, eine teilweise sehr deutliche Verminderung ihrer Aggressivität Artgenossen gegenüber sowie eine deutlich vermindertes Schwanzbeißen bewirkte. So bewirkte die Verabreichung der Guanidinoessigsäure (0,12 Gew.-%) bei Tieren, denen ein Platzangebot von 0,9 m²/Tier zur Verfügung stand, eine Reduktion an verletzten Tieren auf fast die Hälfte. Weiterhin bewirkte die Verabreichung der Guanidinoessigsäure (0,12 Gew.-%) bei Tieren, denen ein Platzangebot von 1,5 m²/Tier zur Verfügung stand, eine Reduktion an verletzten Tieren auf über die Hälfte (vgl. Beispiele, Tabelle 8).

Somit ist auch eine nicht-therapeutische Verwendung oder ein nicht-therapeutisches Verfahren Gegenstand der vorliegenden Erfindung, in der bzw. indem das nicht-therapeutische Mittel während der Aufzucht oder Mast der Schweine verabreicht wird, wobei den Schweinen ein Platzangebot im Bereich von 0,75 bis 2,25 m²/Tier, insbesondere 0,75 bis 1,5 m²/Tier, besonders bevorzugt 0,9 bis 1,5 m²/Tier, zur Verfügung steht.

Die Verabreichung der Guanidinoessigsäure als Mittel, insbesondere als nicht-therapeutisches Mittel, erfolgt gemäß der vorliegenden Erfindung in jedem Fall oral. Dabei kann das Mittel als Einzeldosis oder gemeinsam mit einem Futtermittel für die Schweine verabreicht werden.

Als Futtermittel eignen sich dabei bevorzugt Weizen, Gerste, Triticale, Sojaextraktionsschrot, Rapsextraktionsschrot und Körnermais. Es können aber auch andere Arten von Futtermitteln wie Maiskornsilage, Ackerbohnen, Erbsen, Kleien, Trockenschnitzel, Rapskuchen, Sojabohnen, Sonnenblumenxtraktionsschrot, Zuckerrübenschnitzel eingesetzt werden. In die Futtermittelmischung können zum Erzielen der gewünschten Zusammensetzung Faserträger wie Obsttrester, Sojaschalen, Weizenkleie und Öle bzw. Ölsaaten aus Soja, Raps und Sonnenblumen, Koppelprodukte der Lebensmittelproduktion und Mineralien und Aminosäuren eingearbeitet werden.

Besonders vorteilhaft kann somit eine Verabreichung erfolgen, wenn das nicht-therapeutische Mittel gemeinsam mit einem Futtermittel für die Schweine verabreicht wird und das Futtermittel gewählt wird aus der Gruppe Weizen, Gerste, Triticale, Sojaextraktionsschrot, Rapsextraktionsschrot und Körnermais.

Die Energieinhalte der Futtermittel können breit gewählt werden. Das Futtermittel kann bevorzugt eine Metabolische Energie im Bereich von 10 MJ/kg bis 16 MJ/kg aufweisen. Mit steigender Energiekonzentration reduziert sich die zu applizierende Futtermenge oder es erhöht sich die Wachstumsrate. Vorteilhafte Ausführungen ergeben sich bei Einhaltung der von der bayerischen Landesanstalt für Landwirtschaft (LfL) ausgesprochenen Empfehlungen in "Futterberechnung für Schweine", 23. unveränderte Auflage, Januar 2020. Basis der Futtermittelauswahl und Zusammenstellung bilden die Weender Futttermittelanalysen und Energieschätzgleichungen. In der genannten Schrift sind unter anderem Versorgungsempfehlungen und Richtwerte für Jungsauenaufzucht, Ferkelfütterung und Mastschweinefütterung in Abhängigkeit vom Alter der Tiere genannt. Diese Empfehlungen decken ein breites Band an Parametern ab, wie die Metabolisierbare Energie, Rohprotein, Lysin und praecaecal verdauliches Lysin, Methionin und Cystein, Tryphtophan und Threonin, Rohfaseranteil, Calzium, verdaulicher Phosphor und Natrium.

Im Bereich der Mastschweinefütterung sind Energiekonzentrationen von 13 MJ/kg bis 13,6 MJ/kg vorteilhaft für ein optimales Mastergebnis. Die Wirkung von Guanidinoessigsäure ist nicht abhängig vom Energieinhalt des Futters und von der bereitgestellten Energiemenge solange es zu keinen Mangelerscheinungen kommt. Daher ist die Einhaltung der Empfehlungen vorteilhaft.

Typische Analysenwerte für verschiedene Arten von Getreide und Getreidenebenprodukte, typische Eiweißfutter, Kartoffel- und Nebenprodukte und Rüben- und Rübennebenprodukte aber auch von Grünfutter, Silagen, Heu und Stroh, Brauerei- und Molkereiprodukten und weiteren Nebenprodukten der Lebensmittelverarbeitung, Öle und Ölsaaten sowie von Additiven wie Aminosäuren und Mineralien sind ebenfalls genannt. Vorteilhaft werden die am jeweiligen Standort verfügbaren Futtermittel in der Art und Weise kombiniert, dass die Zusammensetzung des Endfutters den vorher genannten Empfehlungen entspricht.

Guanidinoessigsäure kann in Abhängigkeit vom Fütterungssystem den Tieren verabreicht werden. Generell kann Guanidinoessigsäure mit dem Futter oder mit dem Wasser verabreicht werden. Bei Verabreichung mit dem Futter kann Guanidinoessigsäure mit dem fertigen Futter gemischt werden. Vorteilhaft ist jedoch, dass bei der Herstellung der Futtermittelmischung das Guanidinoessigsäure zunächst zu dem Basisfutter, d.h. dem mengenmäßigen Hauptanteil des Futters gegeben wird und dann die weiteren Futtermittelbestandteile zugegeben werden. Dies hat den Vorteil, dass keine zusätzliche Mischzeit benötigt wird. Bei Verabreichung mit dem Wasser wird in vorteilhafter Weise eine Verabreichung als Salz, beispielsweise als Na- oder Ca-Salz vorgenommen.

Im Allgemeinen ist eine Verabreichung mit dem Futter vorzuziehen, da so eine gleichmäßigere Aufnahme von Guanidinoessigsäure bei Tieren erreicht wird. Die bevorzugte Dosierungsmenge bezieht sich auf das Futtermittel.

Eine Dosierung in das Wasser ist dann vorteilhaft, wenn eine Guanidinoessigsäure-freie Futtermittelmischung vorhanden ist und das Guanidinoessigsäure in einfacher Art und Weise dosiert werden muss. Hierbei ist jedoch der Wasserbedarf der Tiere in Abhängigkeit vom Alter und von der Temperatur zu berücksichtigen, um eine angemessene Dosierung zu erreichen.

Bei der Trockenfütterung ohne Wasser, bei der Trockenfütterung mit Wasser und bei der Trockenfütterung am Breifutterautomaten kann die Dosierung sowohl in das Wasser als auch in das Futtermittel erfolgen. Nur für den Fall, dass die entsprechende Stallung auch andere Inhaltsstoffe mit dem Trinkwasser verabreicht, ist auch die Dosierung von Guanidinoessigsäure in das Wasser angebracht. Ansonsten hat es sich als einfacher erwiesen, Guanidinoessigsäure in Kombination mit dem Futter zu verabreichen.

Bei der Flüssigfütterung kann Guanidinoessigsäure in das flüssige Futter dosiert werden und es wird durch einfaches Rühren homogen verteilt.

Bezogen auf die feste Futtermittelmenge kann eine Guanidinoessigsäure-Menge von 0,02 Gew.-% bis 1 Gew.-%, bevorzugt von 0,05 Gew.-% bis 0,2 Gew.-% dem Futtermittel zugeben werden.

Die Guanidinoessigsäure kann dabei dem Futtermittel in unterschiedlichsten Feststoffzubereitungen oder als Lösung beigemengt werden. Besonders vorteilhaft kann die Guanidinoessigsäure dem Futtermittel in Form eines Granulates, Extrudates oder als Lösung beigemengt werden.

Sollte die Guanidinoessigsäure als Feststoff oder Feststoffzubereitung verwendet werden kann die Guanidinoessigsäure auch auf ein Trägermaterial aufgebracht sein.

Sollte die Guanidinoessigsäure als Einzeldosis verabreicht werden so kann Guanidinoessigsäure bevorzugt in einer Dosis von 4 bis 500 mg pro Tag pro kg Tier, weiter bevorzugt von 10 bis 200 mg pro Tag pro kg Tier, ganz besonders bevorzugt von 20 bis 100 mg pro Tag pro kg Tier verabreicht werden.

Die nachfolgenden Beispiele sollen das Wesen der Erfindung näher erläutern.

### Figurenbeschreibung:

Figur 1: Skizze zur Belegung der Abteile 1 und 4
Figur 2: Skizze zur Belegung der Abteile 2 und 5
Figur 3: Skizze zur Belegung der Abteile 3 und 6

Der Abstand der Boxen mit 14,4 m² zu den Boxen mit 24 m², welcher durch das Versetzen der Gitterstäbe entstand, ist nicht dargestellt.

### Beispiele

### a) Versuchsbeschreibung:

Insgesamt kamen 1440 Schweine (Masttiere der Rasse Piétrain x Danbred) zum Einsatz.

### b) Fütterung:

Ab dem 10. Lebenstag bis zum 27. Lebenstag wurde mit Bonimal SB LiquidStart in Darreichung als Flüssigfütterung in WEDA-Nutrixanlagen beigefüttert. Inhaltsstoffe It. Hersteller: Energie (ME) 15,6 MJ, Rohprotein 21,00 %, Lysin 1,36 %, Rohfaser 0,90 %, Calcium 0,40 %, Phosphor 0,60 %, Zusatzstoffe Vitamin A 25.000 I.E., Vitamin D3 5.000 I.E., Vitamin E 150 mg.

Direkt im Anschluss ab dem 28. Lebenstag bis zum 41. Lebenstag wurde mit Bonimal SB Safe Absetzer 140 gefüttert. Inhaltsstoffe It. Hersteller: Energie (ME) 14,3 MJ, Rohprotein 17,50 %, Lysin 1,14 %, Rohfaser 3,70%, Calcium 0,50 %, Phosphor 0,57 %, Zusatzstoffe Vitamin A 16.000 I.E., Vitamin D3 2.000 I.E., Vitamin E 75 mg.

Ab dem 42. Lebenstag bis zum 60. Lebenstag erfolgte die Fütterung mit Bonimal SK Ferkel 138. Inhaltsstoffe It. Hersteller: Energie (ME) 13,8 MJ, Rohprotein 17,00 %, Lysin 1,25 %, Rohfaser 4,00%, Calcium 0,70 %, Phosphor 0,53 %, Zusatzstoffe Vitamin A 15.000 I.E., Vitamin D3 2.000 I.E., Vitamin E 100 mg.

Ab dem 61. Lebenstag bis zum 90. Lebenstag erfolgte die Fütterung mit Bonimal SK Ferkel 134. Inhaltsstoffe It. Hersteller: Energie (ME) 13,4 MJ, Rohprotein 17,00 %, Lysin 1,20 %, Rohfaser 3,50%, Calcium 0,70 %, Phosphor 0,50 %, Zusatzstoffe Vitamin A 15.000 I.E., Vitamin D3 2.000 I.E., Vitamin E 100 mg.

Ab dem 91. Lebenstag begann der Versuch mit der unterschiedlichen Dosierung von unterschiedlichen Mengen Guanidinoessigsäure in das Futtermittel.

### c) Haltung der Tiere:

Nach einer Säugezeit von 28 Tagen erfolgte eine zweiphasige Ferkelaufzucht bis zum 60. Lebenstage im Flatdeck (0,50 m² pro Ferkel) in Buchten zu 24 Ferkeln.

Die Abferkelställe hatten eine Größe von 5 m². Davon war 1 m² beheizbar mit Ferkelliegeplatten und 4 m² mit Betonspaltenböden mit 11 mm Spaltenweite und 50 mm Auftrittsbreite. Das Ergänzungsfutter wurde seitlich an zwei Wänden in einer WEDA-Nutrixanlage bereitgestellt.

Nach 28 Lebenstagen wurden die Ferkel in das Flatdeck mit einer Spaltenbreite von 14 mm übersiedelt. Die Größe jedes Flatdecks betrug 3 m x 4 m. Futter mit gleichzeitigem Zugang zu Wasser wurde in Breifutterautomaten angeboten. Die Breite des Fressplatzes betrug 18 cm. In jedem Flatdeck waren 2 Beißhölzer für Ferkel angebracht. Stroh war durch einen Strohkorb den Ferkeln zugänglich.

Nach 60 Lebenstagen wurden 1152 Tiere für den Fortgang des Versuches selektiert: Hierbei wurden auffällig schwache Tiere und Tiere mit leicht erkennbaren Verletzungen an Schwanz oder anderen Körperteilen nicht verwendet.

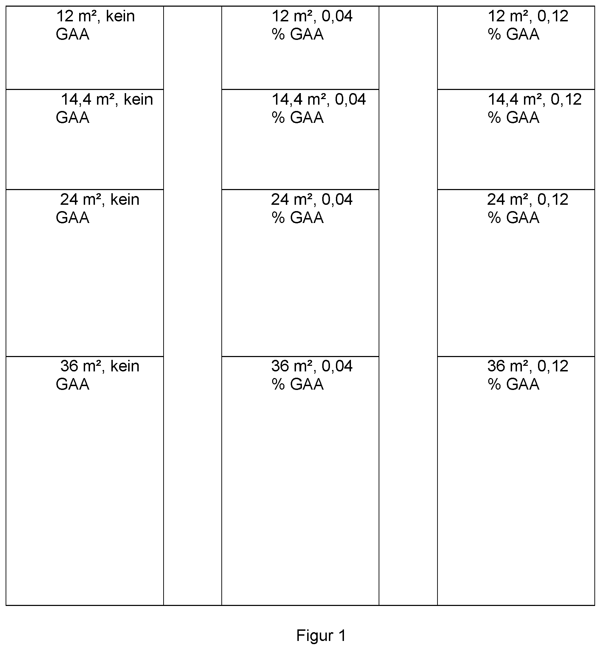

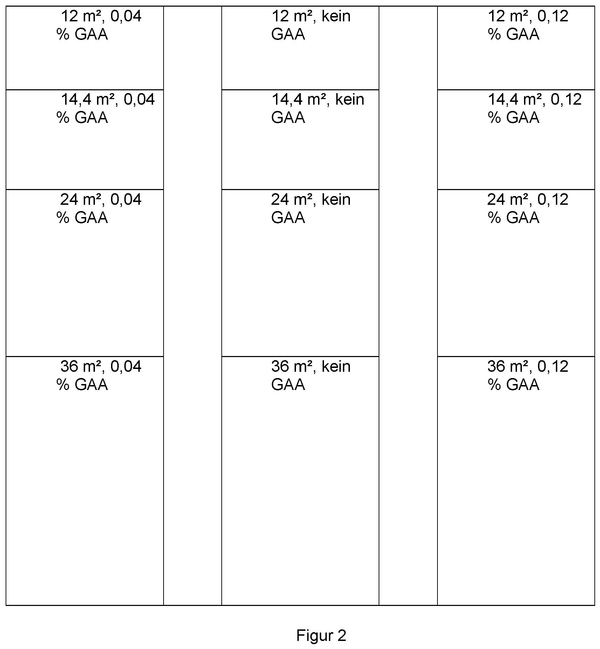

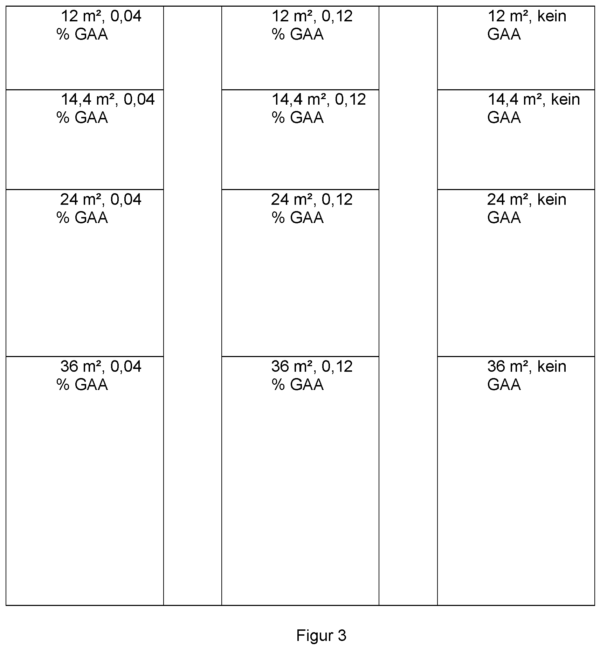

In der anschließenden Mast wurden die Schweine in 72 Buchten zu je 16 Tieren auf Betonspalten ohne Einstreu gehalten. Eine Skizze zum Versuchsaufbau ist mit den Figuren 1 bis 3 dargestellt. Die Spaltenweite betrug 18 mm bei einer Auftrittsbreite von 80 mm. Gefüttert wurde ad libitum über Breifutterautomaten, Typ RM 05 der Firma IBO Stalltechnik GmbH. Die Futtervorlage erfolgte über mehliges Futter.

Die Fressplatzbreite pro Tier betrug 33 cm. Als Beschäftigungsmaterial waren in allen Buchten jeweils vorhanden: 2 hängende Beißhölzer natur für Sauen, 2 Knabberkugeln 5,5 cm Durchmesser. Stroh war durch einen Strohkorb zugänglich. An zwei Wänden waren Schurr Schweinebürsten montiert.

Pro Versuchsparameter wurden 6 Buchten gemischtgeschlechtlich aufgestallt. Für eine gleichmäßige Verteilung wurden die Tiere zunächst in 4 Hauptgruppen 1, 2, 3 und 4 zu je 288 Tieren geteilt. Anschließend wurde von jeder Hauptgruppe eine Unterteilung in 3 Untergruppen A, B und C (UG A, UG B, UG C) zu je 96 Tieren vorgenommen, wobei von jeder Untergruppe die Tiere in 6 Buchten getrennt wurden. Es gab also 12 Versuchsparameter mit je 6 Replikationen und 16 Tieren pro Replikation, entsprechend 1152 Tieren.

Die 72 Buchten waren auf 6 Abteile in einem Stallgebäude aufgeteilt. Je Abteil waren alle 4 Hauptgruppen vertreten, wobei jeder Hauptgruppe eine Buchtengröße zugewiesen wurde (vgl. Tabelle 1). Die Buchtengrößen der Hauptgruppen 3 und 4 wurden durch Entfernen von Gittern aus der Standardbucht von 12 m² realisiert. Zur Realisierung der Buchtengröße von Hauptgruppe 2 wurde ein Teil einer 24 m²-Bucht durch ein Gitter verkleinert. Die Tiere der Hauptgruppen 1, 2, 3 und 4 wurden ohne weiteres Umstallen mit den in der Tabelle 1 aufgeführten Buchtengrößen bis zur 30. Lebenswoche gehalten.

**Tabelle 1: Buchtengröße und verfügbare Fläche pro Tier**

| Hauptgruppe | Buchtengröße | Verfügbare Fläche pro Tier |
|---|---|---|
| 1 | 12 m² | 0,75 m²/Tier |
| 2 | 14,4 m² | 0,9 m²/Tier |
| 3 | 24 m² | 1,5 m²/Tier |
| 4 | 36 m² | 2,25 m²/Tier |

Die verfügbare Fläche pro Tier wurde in Anlehnung an das Staatliche Tierwohllabel für Schweine in Deutschland gewählt. Für Tiere zwischen 50 bis 110 kg gelten hierbei folgende Regelungen: Mindeststandard: 0,75 m²/Tier; Stufe 1: 0,9 m²/Tier; Stufe 2: 1,1 m²/Tier; Stufe 3: 1,5 m²/Tier (davon 0,5 m² Auslauf).

Hauptgruppe 1 entspricht demnach dem gesetzlichen bundesdeutschen Mindeststandard, Hauptgruppe 2 entspricht der Stufe 1 der Staatlichen Tierwohlkennzeichnung im Flächenbedarf, Hauptgruppe 3 entspricht der Stufe 3 der Staatlichen Tierwohlkennzeichnung im Flächenbedarf jedoch in Stallhaltung ohne Auslauf. Den Tieren in Hauptgruppe 4 wurde deutlich mehr Platz geboten als die bislang höchste Stufe der staatlichen Tierwohlkennzeichnung vorsieht.

Die Fütterung ab dem 91. Lebenstag erfolgte in 2 Phasen, welche über alle 4 Hauptgruppen hinweg identisch war.
Phase I: ab dem 91. Lebenstag bis zum 120. Lebenstag
Phase II: ab dem 121. Lebenstag

Für die Untergruppen B und C wurde Guanidinoessigsäure in das Futter zugegeben in einer Menge von 0,04 Gew.-% respektive 0,12 Gew.-% relativ zum festen Futter. Die Mischung erfolgte in einem HIMEL Kompaktmischer FM, Fassungsvermögen 560 kg indem jeweils das Futter vorgelegt wurde und Guanidinoessigsäure als Pulver zugegeben wurde und für 15 min auf höchster Leistungsstufe gemischt wurde.

Die kalkulierten Gehalte an Energie, Rohprotein und Aminosäuren je kg des eingesetzten Futters (880 g TM) sind in Tabelle 2 dargestellt.

**Tabelle 2: Kalkulierte Gehalte und metabolische Energie des Futters**

| | | Phase I | Phase II |
|---|---|---|---|
| | | 91.-120. Lebenstag | > 120. Lebenstag |
| Umsb. Energie (ME) | MJ | 13,2 | 13,0 |
| Rohprotein | g | 155 | 140 |
| Lysin | g | 9,1 | 7,6 |
| Methionin + Cystin | g | 5,9 | 5,4 |
| Threonin | 9 | 6,2 | 5,5 |
| Thryptophan | g | 1,9 | 1,7 |
| Isoleucin | g | 5,2 | 4,8 |
| Valin | g | 6,4 | 6,0 |

Jedes Stallabteil wird von drei Rohrkettenförderern durchzogen, welche jeden Futterautomaten befüllen können. Durch entsprechendes Öffnen oder Schließen von Schiebern kann voreingestellt werden, über welche Rohrkette ein Futterautomat befüllt wird. Somit können in einem Abteil bis zu drei verschiedene Futter vorgelegt werden.

In Tabelle 3 ist das Versuchsregime dargestellt (vgl. auch Figuren 1 bis 3).

**Tabelle 3: Versuchsregime**

| Hauptgruppe | Verfügbare Fläche [m²/Tier] | | Kein GAA | 0,04 Gew.-% GAA | 0,12 Gew.-% GAA |
|---|---|---|---|---|---|
| | | | UGA | UG B | UG C |
| 1 | 0,75 | | A1 | B1 | C1 |
| 2 | 0,9 | | A2 | B2 | C2 |
| 3 | 1,5 | | A3 | B3 | C3 |
| 4 | 2,25 | | A4 | B4 | C4 |

Die Bonitierung erfolgte bei der Remontierung des Bestandes anhand der in Tabelle 4 dargestellten Klassifizierung.

**Tabelle 4: Bonitur**

| | |
|---|---|
| 1 | Ohne Auffälligkeiten |
| 2 | Trockene Bissspuren, nicht blutend |
| 3 | Blutige Bisspuren, mittelgradige Entzündungen |
| 4 | (Teil)-Verlust, hochgradige Entzündungen, große blutende Wunden |

Pro Versuchsregime kam es zu maximal 2 Ausfällen während der Mastphase. Hier wurde die Bonitur zum Zeitpunkt des Ausfalls bewertet.

### Ergebnisse

Wesentlich für eine Bewertung des Bestandes ist das Verhältnis von unverletzten oder nur leicht verletzten Tieren zu mittel- und schwerverletzten Tieren. Da trockene, nichtblutige Bissspuren durch einen spielerischen nichtaggressiven Akt zustande kommen, wurden die Tiere mit Bonitur 2 zu den nicht-verletzen Tieren hinzugerechnet. Als schädlich für das Tierwohl werden Schädigungen ab der Bonitur 3, d.h. blutige Bissspuren, angesehen, die aus aggressiven und nicht mehr spielerischen Handlungen resultieren. Daher wurden für eine Auswertung die Zahlen für die Bonituren 3 und 4 addiert und der prozentuale Anteil dieser Tiere relativ zur Gesamtzahl der jeweiligen Versuchsgruppe ausgewertet.

**Tabelle 5: Gesamtergebnis: Anzahl der Tiere mit den entsprechenden**

| Bonituren bei den jeweiligen Versuchsparametern | | | | | |
|---|---|---|---|---|---|
| Bonitur → | 1 | 2 | 3 | 4 | Summe Bonitur 3 + 4 |
| Versuch ↓ | | | | | |
| A1 | 39 | 42 | 12 | 3 | 15 |
| A2 | 41 | 41 | 11 | 3 | 14 |
| A3 | 43 | 42 | 9 | 2 | 11 |
| A4 | 46 | 43 | 6 | 1 | 7 |
| B1 | 41 | 41 | 10 | 4 | 14 |
| B2 | 50 | 36 | 8 | 2 | 10 |
| B3 | 54 | 36 | 5 | 1 | 6 |
| B4 | 55 | 35 | 6 | 0 | 6 |
| C1 | 40 | 43 | 10 | 3 | 13 |
| C2 | 52 | 36 | 6 | 2 | 8 |
| C3 | 56 | 35 | 4 | 1 | 5 |
| C4 | 55 | 35 | 5 | 1 | 6 |

### A) Betrachtung des Einflusses der zur Verfügung stehenden Fläche pro Tier

Jeweils 288 Tiere wurden bei den jeweiligen Flächen, nämlich 0,75 m²/Tier, 0,9 m²/Tier, 1,5 m²/Tier und 2,25 m²/Tier, gehalten. Hierbei wurden alle Tiere in die Auswertung mit einbezogen, unabhängig davon, ob sie keine Guanidinoessigsäure oder 0,04 Gew.-% Guanidinoessigsäure oder 0,12 Gew.-% Guanidinoessigsäure in das Futter verabreicht bekamen.

**Tabelle 6: Ergebnis Platzangebot**

| Verfügbare Fläche [m²/Tier] | Versuche | Anzahl Tiere Bonitur 3 oder 4 | Verletzte Tiere [%] |
|---|---|---|---|
| 0,75 | A1 + B1 + C1 | 42 | 14,6 |
| 0,9 | A2 + B2 + C2 | 33 | 11,1 |
| 1,5 | A3 + B3 + C3 | 22 | 7,6 |
| 2,25 | A4 + B4 + C4 | 19 | 6,6 |

Wie aus obiger Tabelle 6 hervorgeht, verringern sich mit Zunahme des Platzangebotes die Anzahl und die prozentuale Zahl der verletzten Tiere. Ohne an die Theorie gebunden zu sein, kann vermutet werden, dass bei zu wenig Bewegungsfreiheit pro Tier deren Aggression gefördert wird und es so zu dem Effekt des Schwanzbeißens kommt. Desweitern schafft eine große räumliche Nähe deutlich mehr Möglichkeiten zu Tier-Tier-Interaktionen.

### B) Betrachtung des Einflusses der Supplementierung mit

### Guanidinoessigsäure ohne Berücksichtigung der zur Verfügung stehenden Fläche pro Tier:

Jeweils 384 Tiere wurden bei den verschiedenen Supplementierungsraten mit Guanidinoessigsäure, nämlich ohne Supplementierung, mit 0,04 Gew.-% Supplementierung und mit 0,12 Gew.-% Supplementierung gehalten. Hierbei wurden alle Tiere in die Auswertung mit einbezogen, unabhängig von der jeweils zur Verfügung stehenden Fläche pro Tier.

**Tabelle 7: Ergebnis Supplementierung Guanidinoessigsäure**

| GAA im Futter [Gew.-%] | Versuche | Anzahl Tiere Bonitur 3 oder 4 | Verletzte Tiere [%] |
|---|---|---|---|
| 0 | A1 + A2 + A3 + A4 | 47 | 12,2 |
| 0,04 | B1 + B2 + B3 + B4 | 36 | 9,4 |
| 0,12 | C1 + C2 + C3 + C4 | 32 | 8,3 |

Wie aus obiger Tabelle 7 hervorgeht, verringert sich die Zahl und der prozentuale Anteil verletzter Tiere bei der Supplementierung mit Guanidinoessigsäure. Schon bei einer geringen Supplementierung, nämlich mit 0,04 Gew.-% Guanidinoessigsäure, tritt eine deutliche Reduktion von 12,2 % verletzten Tieren ohne Supplementierung auf 9,4 % verletzten Tieren bei Supplementierung mit 0,04 Gew.-% Guanidinoessigsäure auf. Bei einer Erhöhung der supplementierten Menge Guanidinoessigsäure von 0,04 Gew.-% auf 0,12 Gew.-% verringert sich der Anteil der verletzten Tiere von 9,4 % weiter auf 8,3 %.

### C) Betrachtung des Einflusses der Supplementierung mit

### Guanidinoessigsäure bei Berücksichtigung der zur Verfügung stehenden Fläche pro Tier:

Jeweils 96 Tiere wurden bei verschiedenen Supplementierungsraten mit Guanidinoessigsäure, nämlich ohne Supplementierung, mit 0,04 Gew.-% Supplementierung und mit 0,12 Gew.-% Supplementierung und bei verschiedenem Flächenangebot pro Tier, nämlich 0,75 m²/Tier, 0,9 m²/Tier, 1,5 m²/Tier und 2,25 m²/Tier, gehalten.

**Tabelle 8: Gesamtbetrachtung Supplementierung Guanidinoessigsäure**

| GAA im Futter [Gew.-%] | Verfügbare Fläche [m²/Tier] | Versuch | Verletzte Tiere [%] |
|---|---|---|---|
| 0 | 0,75 | A1 | 15,6 |
| 0,04 | 0,75 | B1 | 14,6 |
| 0,12 | 0,75 | C1 | 13,5 |
| 0 | 0,9 | A2 | 14,6 |
| 0,04 | 0,9 | B2 | 10,4 |
| 0,12 | 0,9 | C2 | 8,3 |
| 0 | 1,5 | A3 | 11,5 |
| 0,04 | 1,5 | B3 | 6,3 |
| 0,12 | 1,5 | C3 | 5,2 |
| 0 | 2,25 | A4 | 7,3 |
| 0,04 | 2,25 | B4 | 6,3 |
| 0,12 | 2,25 | C4 | 6,3 |

Bei den Versuchen, bei denen den Tieren nur 0,75 m²/Tier zur Verfügung stand, nämlich A1, B1 und C1 verringerte sich bei Supplementierung mit Guanidinoessigsäure der prozentuale Anteil der verletzten Tiere nur geringfügig, nämlich von 15,6 % (ohne Supplementierung) auf 14,6 % (Supplementierung mit 0,04 Gew.-% Guanidinoessigsäure) und auf 13,5 % (Supplementierung mit 0,12 Gew.-% Guanidinoessigsäure).

Bei den Versuchen, bei denen den Tieren 0,9 m²/Tier zur Verfügung stand, nämlich A2, B2 und C2 verringerte sich bei Supplementierung mit Guanidinoessigsäure der prozentuale Anteil der verletzten Tiere deutlich, nämlich von 14,6 % (ohne Supplementierung) auf 10,4 % (Supplementierung mit 0,04 Gew.-% Guanidinoessigsäure) und auf 8,3 % (Supplementierung mit 0,12 Gew.-% Guanidinoessigsäure). Die Anzahl der verletzten Tiere hat sich hierdurch fast halbiert. Besonders groß war der Effekt zwischen den Versuchen ohne Zugabe von Guanidinoessigsäure mit 14,6 % verletzen Tieren und Supplementierung mit 0,04 Gew.-% Guanidinoessigsäure mit 10,4 % verletzten Tieren.

Bei den Versuchen, bei denen den Tieren 1,5 m²/Tier zur Verfügung stand, nämlich A3, B3 und C3 verringerte sich bei Supplementierung mit Guanidinoessigsäure der prozentuale Anteil der verletzten Tiere nur geringfügig, nämlich von 11,5 % (ohne Supplementierung) auf 6,3 % (Supplementierung mit 0,04 Gew.-% Guanidinoessigsäure) und auf 5,2 % (Supplementierung mit 0,12 Gew.-% Guanidinoessigsäure). Die Anzahl der verletzten Tiere konnte bei dieser Versuchsreihe mehr als halbiert werden. Auch hier wurde die stärkste Abnahme zwischen den Versuchen ohne Zugabe von Guanidinoessigsäure mit 11,5 % verletzen Tieren und Supplementierung mit 0,04 Gew.-% Guanidinoessigsäure mit 6,3 % verletzten Tieren beobachtet.

Bei den Versuchen, bei denen den Tieren 2,25 m²/Tier zur Verfügung stand, nämlich A4, B4 und C4 verringerte sich bei Supplementierung mit Guanidinoessigsäure der prozentuale Anteil der verletzten Tiere nur geringfügig, nämlich von 7,3 % (ohne Supplementierung) auf 6,3 % (Supplementierung mit 0,04 Gew.-% respektive 0,12 Gew.-% Guanidinoessigsäure). Der Effekt der Supplementierung war zum einen nur gering, zum anderen ergab die Erhöhung der supplementierten Menge Guanidinoessigsäure von 0,04 Gew.-% auf 0,12 Gew.-% keine Verminderung bei der Anzahl der verletzten Tiere.

Zusammenfassend wird gefolgert, dass durch Zugabe von Guanidinoessigsäure in das Schweinefutter die Anzahl der am Schwanz verletzten Tiere abnimmt. Dieser Effekt ist besonders deutlich bei denjenigen Tieren, denen ein Flächenangebot von 0,9 m²/Tier bzw. 1,5 m²/Tier zur Verfügung stand. Aber auch bei einem Flächenangebot von 0,75 m²/Tier und 2,25 m²/Tier ist der positive Einfluss der Supplementierung von Guanidinoessigsäure auf die Anzahl der am Schwanz verletzten Tiere erkennbar.

Ohne an die Theorie gebunden zu sein wird angenommen, dass bei einem Platzbedarf von 0,75 m²/Tier eine derart hohe Stressbelastung vorliegt, dass eine Supplementierung diese Stressbelastung kaum abbauen kann, da die zur Verfügung stehende Fläche unterhalb des natürlichen Platzbedarfes liegt. Bei einem Platzangebot von 2,25 m²/Tier ist der Abstand der Tiere untereinander so groß, dass es seltener zu Tier-Tier-Interaktionen kommen kann. Die Aggression eines Angreifers kann sich abbauen, bevor das Tier seine Aggression auslebt und dem angegriffenen Tier steht mehr Platz zum Ausweichen zur Verfügung.

Die Supplementierung mit Guanidinoessigsäure verringert demzufolge aggressives Verhalten und Verhaltensstörungen von Schweinen, wie zum Beispiel das Schwanzbeißen und demzufolge die dadurch hervorgerufenen Verletzungen und letztendlich die daraus resultierenden wirtschaftlichen Schäden.

## Patentansprüche

1. Nicht-therapeutische Verwendung von Guanidinoessigsäure als Mittel zur Verminderung der Aggressivität von Schweinen und/oder als Mittel zur Behandlung der Caudophagie bei Schweinen.

2. Nicht-therapeutisches Verfahren zur Behandlung der Caudophagie bei Schweinen **dadurch gekennzeichnet, dass** den Schweinen Guanidinoessigsäure als Mittel zur Verminderung der Aggressivität verabreicht wird.

3. Nicht-therapeutische Verwendung oder nicht-therapeutisches Verfahren nach einem der vorgenannten Ansprüche **dadurch gekennzeichnet, dass** das Mittel während der Aufzucht oder Mast verabreicht wird.

4. Nicht-therapeutische Verwendung oder nicht-therapeutisches Verfahren nach einem der vorgenannten Ansprüche **dadurch gekennzeichnet, dass** das Mittel ab dem 60. Lebenstag der Schweine verabreicht wird.

5. Nicht-therapeutische Verwendung oder nicht-therapeutisches Verfahren nach einem der vorgenannten Ansprüche, **dadurch gekennzeichnet, dass** das Mittel während der Aufzucht oder Mast der Schweine verabreicht wird, wobei den Schweinen ein Platzangebot im Bereich von 0,75 bis 2,25 m²/Tier zur Verfügung steht.

6. Nicht-therapeutische Verwendung oder nicht-therapeutisches Verfahren nach einem der vorgenannten Ansprüche, **dadurch gekennzeichnet, dass** das Mittel gemeinsam mit einem Futtermittel für die Schweine verabreicht wird.

7. Nicht-therapeutische Verwendung oder nicht-therapeutisches Verfahren nach einem der vorgenannten Ansprüche, **dadurch gekennzeichnet, dass** das Futtermittel gewählt wird aus der Gruppe Weizen, Gerste, Triticale, Sojaextraktionsschrot, Rapsextraktionsschrott und Körnermais.

8. Nicht-therapeutische Verwendung oder nicht-therapeutisches Verfahren nach einem der vorgenannten Ansprüche, **dadurch gekennzeichnet, dass** das Futtermittel eine Metabolische Energie im Bereich von
10 bis 16 MJ/kg Futter aufweist.

9. Nicht-therapeutische Verwendung oder nicht-therapeutisches Verfahren nach einem der vorgenannten Ansprüche, **dadurch gekennzeichnet, dass** die Guanidinoessigsäure in einer Dosis von 4 bis 500 mg pro Tag pro kg Tier verabreicht wird.

10. Nicht-therapeutische Verwendung oder nicht-therapeutisches Verfahren nach einem der vorgenannten Ansprüche, **dadurch gekennzeichnet, dass** die Guanidinoessigsäure in einer Menge von 0,02 bis 1 Gew.-%, bevorzugt von 0,05 bis 0,2 Gew.-%, bezogen auf das Futtermittel verabreicht wird.

11. Nicht-therapeutische Verwendung oder nicht-therapeutisches Verfahren nach einem der vorgenannten Ansprüche **dadurch gekennzeichnet, dass** die Guanidinoessigsäure dem Futtermittel in Form eines Granulates, Extrudates oder als Lösung beigemengt wird.

12. Nicht-therapeutische Verwendung oder nicht-therapeutisches Verfahren nach einem der vorgenannten Ansprüche **dadurch gekennzeichnet, dass** die Guanidinoessigsäure auf ein Trägermaterial aufgebracht ist.

## Claims

1. Non-therapeutic use of guanidinoacetic acid as an agent for reducing aggressiveness in pigs and/or as an agent for treating caudophagia in pigs.

2. Non-therapeutic method for treating caudophagia in pigs, **characterized in that** guanidinoacetic acid is administered to the pigs as an agent for reducing aggressiveness.

3. Non-therapeutic use or non-therapeutic method according to any of the preceding claims **characterized in that** the agent is administered during breeding or fattening.

4. Non-therapeutic use or non-therapeutic method according to any of the preceding claims **characterized in that** the agent is administered from the 60th day of life of the pigs.

5. Non-therapeutic use or non-therapeutic method according to any of the preceding claims, **characterized in that** the agent is administered during breeding or fattening of the pigs, wherein the pigs are provided with space in the range of 0.75 to 2.25 m²/animal.

6. Non-therapeutic use or non-therapeutic method according to any of the preceding claims, **characterized in that** the agent is administered together with a feed for the pigs.

7. Non-therapeutic use or non-therapeutic method according to any one of the preceding claims, **characterized in that** the feed is selected from the group consisting of wheat, barley, triticale, soybean extraction meal, rapeseed extraction meal and grain maize.

8. Non-therapeutic use or non-therapeutic method according to any one of the preceding claims, **characterized in that** the feed has a metabolic energy in the range of 10 to 16 MJ/kg of feed.

9. Non-therapeutic use or non-therapeutic method according to any one of the preceding claims, **characterized in that** the guanidinoacetic acid is administered in a dose of 4 to 500 mg per day per kg of animal.

10. Non-therapeutic use or non-therapeutic method according to any one of the preceding claims, **characterized in that** the guanidinoacetic acid is administered in an amount of from 0.02 to 1 wt.-%, preferably from 0.05 to 0.2 wt.-%, based on the feed.

11. Non-therapeutic use or non-therapeutic method according to any one of the preceding claims, **characterized in that** the guanidinoacetic acid is added to the feed in the form of a granulate, extrudate or solution.

12. Non-therapeutic use or non-therapeutic method according to any one of the preceding claims, **characterized in that** the guanidinoacetic acid is applied to a carrier material.

## Revendications

1. Utilisation non thérapeutique de l'acide guanidinoacétique comme agent de réduction de l'agressivité des porcs et/ou comme agent de traitement de la caudophagie chez les porcs.

2. Procédé non thérapeutique de traitement de la caudophagie chez les porcs, **caractérisé en ce que** l'on administre aux porcs de l'acide guanidinoacétique comme agent de réduction de l'agressivité.

3. Utilisation non thérapeutique ou procédé non thérapeutique selon l'une des revendications précédentes, **caractérisé en ce que** l'agent est administré pendant l'élevage ou l'engraissement.

4. Utilisation non thérapeutique ou procédé non thérapeutique selon l'une des revendications précédentes, **caractérisé en ce que** l'agent est administré à partir du 60e jour de vie des porcs.

5. Utilisation non thérapeutique ou procédé non thérapeutique selon l'une des revendications précédentes, **caractérisé en ce que** l'agent est administré pendant l'élevage ou l'engraissement des porcs, ces derniers disposant d'un espace de 0,75 à 2,25 m²/animal.

6. Utilisation non thérapeutique ou procédé non thérapeutique selon l'une des revendications précédentes, **caractérisé en ce que** l'agent est administré conjointement avec un aliment pour les porcs.

7. Utilisation non thérapeutique ou procédé non thérapeutique selon l'une des revendications précédentes, **caractérisé en ce que** l'aliment pour animaux est choisi parmi le groupe comprenant le blé, l'orge, le triticale, le tourteau d'extraction de soja, le tourteau d'extraction de colza et le maïs en grains.

8. Utilisation non thérapeutique ou procédé non thérapeutique selon l'une des revendications précédentes, **caractérisé en ce que** l'aliment présente une énergie métabolique comprise dans la plage de 10 à 16 MJ/kg d'aliment.

9. Utilisation non thérapeutique ou procédé non thérapeutique selon l'une des revendications précédentes, **caractérisé en ce que** l'acide guanidinoacétique est administré à une dose de 4 à 500 mg par jour par kg d'animal.

10. Utilisation non thérapeutique ou procédé non thérapeutique selon l'une des revendications précédentes, **caractérisé en ce que** l'acide guanidinoacétique est administré en une quantité de 0,02 à 1 % en poids, de préférence de 0,05 à 0,2 % en poids, par rapport à l'aliment.

11. Utilisation non thérapeutique ou procédé non thérapeutique selon l'une des revendications précédentes, **caractérisé en ce que** l'acide guanidinoacétique est incorporé à l'aliment pour animaux sous forme de granulés, d'extrudés ou de solution.

12. Utilisation non thérapeutique ou procédé non thérapeutique selon l'une des revendications précédentes, **caractérisé en ce que** l'acide guanidinoacétique est appliqué sur un matériau de support.
